(19) Europäisches Patentamt
European Patent Office
Office européen des brevets

(11) EP 1 592 721 B1

(12) **FASCICULE DE BREVET EUROPEEN**

(45) Date de publication et mention de la délivrance du brevet:
**30.07.2008 Bulletin 2008/31**

(21) Numéro de dépôt: **03816146.9**

(22) Date de dépôt: **12.12.2003**

(51) Int Cl.:
***C08F 265/06*** *(2006.01)* ***A61K 8/72*** *(2006.01)*
***C08F 293/00*** *(2006.01)*

(86) Numéro de dépôt international:
**PCT/FR2003/003697**

(87) Numéro de publication internationale:
**WO 2004/078809 (16.09.2004 Gazette 2004/38)**

(54) **POLYMERES HYPERBRANCHES A BASSE TEMPERATURE DE TRANSITION VITREUSE ET LEURS UTILISATIONS EN COSMETIQUE**

HYPERVERZWEIGTE POLYMERE MIT NIEDRIGER GLASÜBERGANGSTEMPERATUR UND DEREN VERWENDUNG IN DER KOSMETIK

HYPERBRANCHED POLYMERS WITH LOW GLASS-TRANSITION TEMPERATURE AND USES THEREOF IN COSMETICS

(84) Etats contractants désignés:
**AT BE BG CH CY CZ DE DK EE ES FI FR GB GR HU IE IT LI LU MC NL PT RO SE SI SK TR**

(30) Priorité: **31.01.2003 FR 0301147**

(43) Date de publication de la demande:
**09.11.2005 Bulletin 2005/45**

(73) Titulaire: **L'ORÉAL**
**75008 Paris (FR)**

(72) Inventeur: **MOUGIN, Nathalie**
**F-75011 Paris (FR)**

(74) Mandataire: **Dodin, Catherine**
**L'OREAL - D.I.P.I.**
**25-29 Quai Aulagnier**
**92600 Asnières (FR)**

(56) Documents cités:
**WO-A-00/71606**
**WO-A-01/96429**
**WO-A-95/06079**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 592 721 B1

## Description

L'invention a pour objet des polymères hyperbranchés comportant au moins une séquence polymérique ayant une faible température de transition vitreuse (Tg) ainsi que le procédé de fabrication de ces polymères. Elle vise également des compositions cosmétiques contenant ces polymères ainsi que leurs utilisations, en particulier dans le domaine des produits de fixation et/ou de maintien de la coiffure.

On connaît des *"polymères hyperbranchés",* c'est-à-dire des polymères comprenant au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre. Chaque point de ramifications est disposé au coeur d'au moins une chaîne.

Au sens de la présente invention, on entend par *"point de ramification trivalent"*, le point de jonction entre trois branches polymériques, dont au moins deux branches sont de constitution chimique ou de structure différentes. Par exemple, certaines branches peuvent être hydrophiles, c'est-à-dire comporter en majorité des monomères hydrophiles et d'autres branches peuvent être hydrophobes, c'est-à-dire comporter en majorité des monomères hydrophobes. D'autres branches encore peuvent former un polymère statistique ou un polymère bloc.

On préfère les polymères hyperbranchés comprenant au moins trois branches polymériques, formant indifféremment la branche principale ou une branche secondaire, et comportant, chacune, au moins un point de ramification trivalent pour former au moins deux points de ramification trivalents, distincts et indépendants l'un de l'autre. Chaque point de ramification est disposé au coeur d'au moins une chaîne.

Par analogie, on entend par point de *"ramification au moins trivalent"*, les points de jonction entre au moins trois branches polymériques, par exemple n branches polymériques, dont n-1 branches au moins sont de constitution chimique ou de structure différentes.

On entend par *"coeur de chaîne"*, les atomes situés à l'intérieur de la chaîne polymérique, à l'exclusion des atomes formant les deux extrémités de cette chaîne.

La définition conforme à l'invention de la notion de *"polymère hyperbranché"* n'englobe pas, notamment:

- les polymères branchés ou à greffons, c'est à dire les polymères constitués d'une chaîne principale présentant de multiples points de ramifications trivalents dont chacun est le point de départ d'une chaîne latérale linéaire, les chaînes latérales pouvant être constituées de 1 ou plusieurs blocs, ces chaînes latérales étant de nature identique ou différente de la chaîne principale, comme par exemple ceux décrits dans le document EP 815848, relatifs à des copolymères à squelette carboné/fluoré de Tg compris entre 0 et 30°C et à greffons rigides de Tg supérieure à 25°C ou dans le document WO 97/35541, relatif à des copolymères à squelette vinylique/acrylique rigide, hydrophile et à greffons flexibles hydrophobes,

- les polymères en peigne, (cas particulier des polymères à greffons) c'est à dire les polymères constitués d'une chaîne principale présentant de multiples points de ramifications trivalents dont chacun est le point de départ d'une chaîne latérale linéaire (Glossary of basic terms in polymer science / IUPAC/1996*),* les points de ramifications étant situés à intervalles réguliers.

- les polymères étoiles, c'est-à-dire ceux dont tous les points de ramifications se trouvent localisés en un seul point.

La figure 1 annexée est destinée à faciliter la compréhension de la notion de *"polymère hyperbranché"*. Elle montre:

1a: Un premier exemple de structure hyperbranchée,
1b: Un deuxième exemple de structure hyperbranchée,
1c: Une structure hyperbranchée minimale,
1d: Une structure de polymère peigne,
1e: Une structure de polymère branché,
1 f: Une structure de polymère étoile,

Sur les figures, le point "T" représente le point trivalent. Les traits fins, gras et ceux formés par une succession de cercles représentent des branches polymériques, de constitution chimique ou de structure différentes.

Selon l'invention, la mesure de la température de transition vitreuse (Tg) du polymère est effectuée par DMTA (Dynamical and Mechanical Temperature Analysis ou Analyse dynamique et mécanique de température).

Pour mesurer la température de transition vitreuse (Tg) du polymère, on effectue des essais de viscoélasticimétrie avec un appareil DMTA de Polymer laboratories, sur un échantillon de film de polymère d'environ 150 $\pm$ 50 $\mu$m d'épaisseur, 5 mm de largeur et 10 mm de longueur, après séchage pendant 24 heures à 23 °C et à 50-55 % d'humidité

relative. On impose à cet échantillon une sollicitation de traction. L'échantillon subit une force statique de 0,01 N à laquelle se superpose un déplacement sinusoïdal de $\pm$ 8 $\mu$m à la fréquence de 1 Hz. On travaille ainsi dans le domaine linéaire, sous de faibles niveaux de déformation. Cette sollicitation de traction est effectuée sur l'échantillon à des températures variant de -150 °C à + 220 °C, avec une variation de température de 3 °C par minute.

On mesure alors le module complexe E* = E' + iE" du polymère testé en fonction de la température.

De ces mesures, on déduit les modules dynamiques E' de conservation et E" de perte, ainsi que le pouvoir amortissant : tg$\delta$ = E"/E'.

Puis on trace la courbe des valeurs de tg$\delta$ en fonction de la température ; cette courbe présente au moins un pic. La température de transition vitreuse Tg du polymère correspond à la température à laquelle se situe le sommet de ce pic.

Lorsque la courbe présente au moins 2 pics (dans ce cas, le polymère présente au moins 2 Tg), on prend comme valeur de Tg du polymère testé la température pour laquelle la courbe présente le pic de plus forte amplitude (c'est à dire correspondant à la plus grande valeur de tg$\delta$ ; on ne considère dans ce cas que la Tg « majoritaire » comme valeur de Tg du polymère testé).

Des polymères hyperbranchés ainsi que leur procédé de fabrication et leur utilisation en cosmétique capillaire sont, par exemple, décrits dans la demande internationale WO01/96429.

Cette demande internationale WO01/96429 concerne des polymères blocs hyperbranchés, préparés à partir d'un premier type de monomères acryliques et de monomères de branchement possédant deux fonctions polymérisables de réactivités différentes, de façon à obtenir des polymères comportant des motifs allyliques pendants, pouvant être polymérisés ultérieurement, en présence d'un second type de monomères acryliques.

Toutefois, les polymères, décrits dans cette demande antérieure, améliorent trop faiblement la tenue et/ou le maintien de la coiffure.

Dans le domaine de la cosmétique, on cherche souvent à disposer de compositions permettant d'obtenir un dépôt notamment adhésif ou filmogène, sur les matières kératiniques considérées, telles que les cheveux, la peau, les cils ou les ongles.

En particulier, ces compositions peuvent apporter de la couleur (compositions de maquillage ou de coloration capillaire), de la brillance ou de la matité (compositions de soin ou de maquillage de la peau), des propriétés physiques telles que de la mise en forme (compositions capillaires notamment de coiffage), des propriétés de soin ou de protection.

On recherche généralement une bonne rémanence et tenue dans le temps du dépôt cosmétique, ainsi qu'une bonne adhésion sur le support. En particulier, il est souhaitable que ce dépôt puisse résister aux agressions mécaniques telles que frottements, transferts par contact d'un autre objet; à l'eau, à la sueur, aux larmes, à la pluie, au sébum et aux huiles. Ceci est particulièrement vrai en maquillage, notamment dans le domaine des rouges à lèvres où l'on recherche la tenue prolongée de la couleur et de la brillance et le non transfert de la couleur; dans le domaine des fonds de teint, fards à paupière et poudres où l'on recherche la tenue de la couleur apportée, en maintenant le plus longtemps possible la matité de la teinte initiale malgré la sécrétion de sébum et de sueur, ainsi que le non transfert.

En outre, les compositions de maquillage doivent être confortables à porter et ne pas présenter une texture trop collante.

Le problème posé par l'invention est de trouver des polymères procurant aux fibres une cosmétique naturelle et durable et aptes à former un film suffisamment homogène et résistant.

De manière surprenante et inattendue, la Demanderesse a maintenant découvert que certains polymères hyperbranchés, répondant à des impératifs bien spécifiques en termes de température de transition vitreuse (Tg), satisfont pleinement aux exigences indiquées ci-avant.

L'invention a pour objet des polymères hyperbranchés comprenant au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre, chaque point de ramifications étant disposé au coeur d'au moins une chaîne, et comprenant au moins une séquence polymérique dont la température de transition vitreuse (Tg) est comprise entre -150 et -20°C, et de préférence, entre -150 et -30°C, et plus préférentiellement entre -150 et -40°C, la proportion relative en poids de cette séquence polymérique étant supérieure à 50 %, et préférentiellement supérieure à 55% et plus préférentiellement encore supérieure à 60%, par rapport au poids total du polymère.

Sans être lié par la théorie, la Demanderesse pense que la proportion supérieure à 50 % en poids de séquence polymérique présentant une faible Tg est responsable de la formation d'un film pouvant constituer un filet souple autour des cheveux ou un " maillage " souple sur la peau.

De préférence, le polymère selon l'invention comprend, en outre, au moins une séquence polymérique dont la température de transition vitreuse (Tg) est comprise entre 20 et 150°C, et préférentiellement entre 20 et 100°C.

Avantageusement, le polymère selon l'invention comporte des points de réticulation formant entre 0.1 et 10 %, et préférentiellement entre 0.5 et 5% du nombre total des liaisons du polymère.

Le paramètre physique caractérisant les propriétés élastiques des polymères hyperbranché est notamment

leur recouvrance en traction et son élongation à la rupture.

* La recouvrance (R donnée en %) est déterminée par essai de fluage en traction consistant à étirer rapidement une éprouvette jusqu'à un taux d'allongement prédéterminé, puis à relâcher la contrainte et à mesurer la longueur de l'éprouvette. Si la mesure est réalisée juste après l'étirement : il s'agira de la recouvrance instantanée, Ri ou si elle est faite après un temps t , il s'agira de la recouvrance Rt.

* Lors d'essais sous traction, la déformation à la rupture ($\varepsilon_{rupture}$ donnée en %) correspond à la déformation maximale de l'échantillon de copolymère avant le point de rupture.

L'essai de fluage utilisé pour la caractérisation des polymères hyperbranchés à caractère élastique de la présente invention se déroule de la manière suivante :

Tout d'abord, il y a lieu de préciser que ces paramètres concernent un film obtenu par séchage d'une solution du copolymère dans le solvant approprié audit copolymère, par exemple l'eau, à température ambiante et à un taux d'humidité relative proche de 50 %.

On mesure les propriétés mécaniques du film en traction monotone selon la norme ASTM Standards, volume 06.01 D 2370-92 'Standard Test Method for Tensile Properties of Organic Coatings'.
On impose donc une vitesse de déplacement et on mesure simultanément la longueur (L) de l'éprouvette et la force (f) nécessaire pour imposer cette longueur.

On utilise, comme éprouvette, un film du copolymère ayant une épaisseur de 150 $\pm$ 50$\mu$m obtenu après 48 heures de séchage à 23 $\pm$ 2°C et 55 $\pm$ 5% d'humidité relative. Ce film est obtenu à partir d'une solution ou d'une dispersion à 6 % en poids dudit copolymère dans de l'eau ou de l'éthanol, déposée sur une matrice en téflon .
L'éprouvette est de forme haltère, de longueur utile 33 mm et de largeur utile 6 mm.

Les essais sont réalisés sur un appareil de traction équipé d'un extensomètre optique pour la mesure du déplacement et commercialisé sous l'appellation Zwick Z010.

Les mesures sont réalisées dans les mêmes conditions de température et d'humidité que pour le séchage, c'est-à-dire à une température de 23 $\pm$ 2°C et une humidité relative de 55 $\pm$ 5%.

Chaque éprouvette est fixée entre deux mors, distants de 50 $\pm$ 1 mm l'un de l'autre, et est étirée à une vitesse de 50 mm/min. (dans les conditions de température et d'humidité relative ci-dessus)
On impose donc une vitesse de déplacement et on mesure simultanément la longueur (L) de l'éprouvette et la force (f) nécessaire pour imposer cette longueur.
La longueur (L) est mesurée avec un extensomètre optique à l'aide de pastilles adhésives placées sur l'éprouvette haltère. La distance initiale entre ces 2 pastilles définit la longueur utile Lo utilisée pour calculer la déformation $\varepsilon$ = (L/Lo) x100 exprimée en %.

La recouvrance élastique est déterminée comme telle :
La recouvrance élastique (R donnée en %) est déterminée en étirant l'éprouvette jusqu'à un allongement de 100 % ($L_{max}$), c'est-à-dire jusqu'à 2 fois sa longueur initiale. On relâche alors la contrainte en imposant une vitesse de retour égale à la vitesse de traction, soit 50 mm/minute.

La recouvrance élastique instantanée ($R_i = \varepsilon_i$) est déterminée par mesure de l'allongement de l'éprouvette (exprimé en % par rapport à la longueur initiale) immédiatement après retour à charge nulle. Elle est donc définie par la déformation instantanée résiduelle, à contrainte nulle :$\varepsilon_{ir}$.

La recouvrance élastique instantanée ($R_i = \varepsilon_i$) est calculée à l'aide de la formule suivante :

$$R_i = \varepsilon_i = 100 - \varepsilon_{i\ \text{résiduelle}}$$

La valeur de la recouvrance instantanée dépend de nombreux facteurs tels que la nature, le nombre, la disposition et la proportion relative des séquences rigides et souples, ou encore la masse molaire du polymère.

Les polymères hyperbranchés à caractère élastique de la présente invention ont généralement une recouvrance instantanée ($R_i$), mesurée dans les conditions indiquées ci-dessus, comprise entre 50 et 100 % de préférence comprise entre 55% et100% mieux encore entre 55 et 95% et idéalement entre 60et 95%

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300} = R_{300}$). La recouvrance en % à 300 secondes ($R_{300}$) est donnée par la formule ci-après:

$$\varepsilon_{300} = R_{300} = 100 - \varepsilon_{300\ \text{résiduelle}}$$

Les polymères hyperbranchés à caractère élastique de la présente invention ont généralement une recouvrance à 300 secondes ($R_{300}$), mesurée dans les conditions indiquées ci-dessus, comprise entre 55 et 100 %, de préférence comprise entre 60% et 100% mieux encore, entre 80 et 100 %.

Les copolymères de l'invention sont définis également par une déformation ou allongement à la rupture $\varepsilon_{rupture}$ qui est donnée en % et qui correspond à la déformation maximale de l'échantillon de copolymère avant le point de rupture.

Dans ce cas, l'essai est conduit jusqu'à rupture de l'éprouvette.

De plus, le polymère hyperbranché présente, avantageusement, une élongation à la rupture supérieure à 1000%, et plus préférentiellement supérieure à 1500%

Conformément à l'invention, le polymère présente, avantageusement, une solubilité dans l'eau supérieure à 1 % en poids à 20°C.

Un homopolymère est dit hydrosoluble s'il forme une solution limpide lorsqu'il est en solution à 1 % en poids dans l'eau, à 25°C.

Un homopolymère est dit hydrodispersible si, à 1% en poids dans l'eau, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Avantageusement, le polymère hyperbranché selon l'invention comprend des motifs dérivés d'un ou de plusieurs monomères éthyléniques capables en particulier de polymériser par voie radicalaire.

Plus avantageusement encore, les motifs dérivés d'un ou de plusieurs monomères éthyléniques sont choisis parmi les acides carboxyliques ou sulfoniques tel que l'acide acrylique ou méthacrylique, les (méth)acrylates d'alkyle en $C_{1-20}$ à chaîne linéaire, ramifiée ou cyclique ou hétérocyclique, les (méth)acrylates d'hydroxyalkyle en $C_{1-4}$, certains esters de vinyle, certains éthers de vinyle, le styrène, certains styrènes substitués, les monomères hétérocycliques, les (méth)acrylates de mono-, di- ou poly(éthylèneglycol) à extrémité hydroxyle éventuellement éthérifiée, le (méth)acrylamide, certains méthacrylamides aliphatiques, cycloaliphatiques ou aromatiques , les monomères (méth)acrylates ou vinyliques à groupe fluoré ou perfluoré ou les (méth)acrylamides à groupe fluoré ou perfluoré, les monomères (méth) acrylates ou vinyliques siliconés ou les (méth)acrylamides siliconés, les monomères acryliques ou vinyliques comportant une fonction amine éventuellement neutralisée ou quaternisée, et les dérivés carboxybétaïnes ou sulfobétaïnes éthyléniques.

De préférence, la masse moléculaire moyenne en nombre du copolymère est comprise entre 5000 g/mol et 1 500 000 g/mol, notamment entre 5500 g/mol et 1000 000 g/mol, et encore mieux entre 6000 g/mol et 900 000 g/mol.

Plus préférentiellement, le polymère selon l'invention comprend des motifs hydrophiles, dont la proportion en poids est comprise entre 5 et 70%, de préférence entre 5 et 65% et plus préférentiellement entre 5 et 50%, ces motifs hydrophiles étant, en particulier, dérivés d'un ou de plusieurs monomères éthyléniques.

On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique).

Les polymères hyperbranchés selon l'invention peuvent être obtenus à partir de procédés de polymérisation en eux-mêmes connus de l'homme de l'art.

Ces procédés peuvent notamment comprendre au moins une étape consistant en une polymérisation radicalaire, par exemple avec des agents multifonctionnels. Dans ce cas, on peut faire réagir:

- des monomères éthyléniques polymérisables par voie radicalaire, comportant des insaturations, comme des monomères de type (méth)acrylate, (méth)acrylamide, vinylique ou allylique, et
- des composés multifonctionnels, c'est-à-dire comportant de multiples insaturations, en proportion comprise environ entre 0,05 à 15%, et mieux de 0,05 à 12%, et préférentiellement de 0,05% à 10% en poids par rapport au poids total du composé.

De façon préférentielle, les fonctions du composé multifonctionnel présentent des réactivités différentes. Par exemple, le composé multifonctinnel peut comporter une fonction acrylate et une fonction allylique.

La synthèse de polymères hyperbranchés peut aussi être réalisée de la façon suivante : On peut faire réagir:

- des monomères éthyléniques polymérisables par voie radicalaire, comportant des groupements (méth)acrylate, (méth)acrylamide, vinylique ou allylique, schématisés par
  =---R

- des composés schématisés par =---A comportant, outre l'insaturation, un site capable d'amorcer la polymérisation, après activation par la température, le rayonnement UV ou selon tout autre mécanisme différent du mécanisme de polymérisation de l'insaturation ,

lesquels sont donc des composés comportant à la fois un site capable de promouvoir la propagation des chaînes c'est à dire comportant une double liaison ( = ) et un site A capable d'amorcer les chaînes.
Cette méthode est nommée "self condensing vinyl polymerization". Elle est décrite dans l'ouvrage « chimie et physico chimie des polymères » M Fontanille, Y Ganou DUNOD 2002, page 368.

Pour former des polymères hyperbranchés, les insaturations ou/et le site A apte à amorcer les chaînes peuvent être capables de réagir selon un mécanisme de polymérisation radicalaire contrôlée. En particulier selon un procédé de polymérisation par transfert d'atome (ATRP), décrit notamment dans macromolecules 2002 ,35,1146-48 Jho JY et Yoo SH; Polym prepr 1996, 37(2), 413-14; Matyjaszewski progress in polymer science26 (3),337-77, 2001 Annexe 2.2; Chem review 2001, 101/12,3737 ; Mueller macromolecules 34/18,62026-13, 2001.

D'une manière générale, la polymérisation radicalaire par transfert d'atomes s'effectue par polymérisation d'un ou de plusieurs monomères polymérisables par voie radicalaire, en présence :

- d'un amorceur ayant au moins un atome d'halogène transférable,
- d'un composé comprenant un métal de transition susceptible de participer à une étape de réduction avec l'amorceur et une chaîne polymérique "dormante" ;
- et d'un ligand pouvant être choisi parmi les composés comprenant un atome d'azote (N), d'oxygène (O), de phosphore (P) ou de soufre (S), susceptibles de se coordonner par une liaison audit composé comprenant un métal de transition.

Ce procédé est en particulier décrit dans la demande WO 97/18247 et dans l'article de Matyjasezwski et al. publié dans JACS, 117, page 5614 (1995).

L'atome d'halogène est de préférence un atome de chlore ou de brome.

Dans ce cas, le site A sera par exemple R1--C1 avec R1= benzene, OCOCH(CH3)-

Sans être exhaustif, d'autres procédés utilisables est la méthode des nitroxydes, décrite par exemple dans Chem review 2001,101(12) p 3681,

La technique de polymérisation radicalaire par réaction avec un nitroxyde consiste à bloquer l'espèce radicalaire en croissance sous forme de liaison de type C-O $NR_1R_2$, $R_1$ et $R_2$ pouvant être, indépendamment l'un de l'autre, un radical alkyle ayant de 2 à 30 atomes de carbone ou formant l'un et l'autre, avec l'atome d'azote, un cycle ayant de 4 à 20 atomes de carbone, comme par exemple un cycle 2,2,6,6-tétraméthylpipéridinyle.

Cette technique de polymérisation est notamment décrite dans les articles "Synthesis of nitroxy-functionalized polybutadiène by anionic polymerization using a nitroxy-functionalized terminator", publié dans Macromolecules 1997, volume 30, pages 4238 - 4242, et "Macromolecular engineering via living free radical polymerizations" publié dans Macromol. Chem. Phys. 1998, vol. 199, pages 923 - 935, ou bien encore dans la demande WO-A-99/03894.
dans ce cas, le site A sera par exemple :

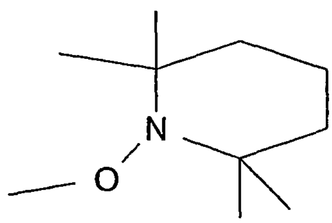

On peut aussi utiliser:

- la méthode polymérisation anionique, décrite, par exemple dans chem review2001, 101/12 , 3787,
- la méthode polymérisation cationique, décrite, par exemple dans Charleux B;R Faust, Advances in polyme science 142,p1-69,1999.

De préférence, le procédé a lieu en deux étapes de façon à générer des séquences de natures différentes.

Avantageusement, on effectue la polymérisation radicalaire à partir de monomères éthyléniques.

L'invention a également pour objet une composition cosmétique, caractérisée par le fait qu'elle comprend, dans un milieu cosmétiquement acceptable, au moins un polymère hyperbranché comprenant au moins trois branches polymériques, formant indifféremment la branche principale ou une branche secondaire, et comportant, chacune, au

moins un point de ramification au moins trivalent pour former au moins deux points de ramification au moins trivalents, distincts et indépendants l'un de l'autre, chaque point de ramifications étant disposé au coeur d'au moins une chaîne, et ce polymère comprend au moins une séquence polymérique dont la température de transition vitreuse (Tg) est comprise entre -150 et -20°C, et de préférence, entre -150 et -30°C, et plus préférentiellement entre -150 et -40°C, la proportion relative en poids de cette séquence polymérique étant supérieure à 50 %, et préférentiellement supérieure à 55% et plus préférentiellement encore supérieure à 60%, par rapport au poids total du polymère.

Les compositions conformes à l'invention peuvent former un dépôt filmogène, notamment sur les cheveux.

Avantageusement, dans les compositions selon l'invention, le polymère hyperbranché comprend, en outre, au moins une séquence polymérique dont la température de transition vitreuse (Tg) est comprise entre 20 et 150°C. Plus avantageusement, le polymère hyperbranché présente une recouvrance instantanée comprise entre 60 et 100 %, et plus avantageusement encore, le polymère hyperbranché possède une recouvrance à 300 secondes comprise entre 80 et 100 %.

De préférence, dans les compositions selon l'invention, le polymère hyperbranché présente une élongation à la rupture supérieure à 1500%.

Plus préférentiellement, le polymère hyperbranché présente une solubilité dans l'eau supérieure à 5 % en poids à 20°C.

Plus préférentiellement, le polymère hyperbranché comporte un motif hydrophile, dont le pourcentage en poids par rapport au poids total du polymère est compris entre 5 et 70%, de préférence entre 5 et 55%, et plus préférentiellement encore entre 5 et 50%, dérivés d'un ou de plusieurs monomères éthyléniques.

Encore plus préférentiellement, le polymère hyperbranché comprend des motifs dérivés d'un ou de plusieurs monomères éthyléniques.

Plus avantageusement encore, parmi les motifs dérivés d'un ou de plusieurs monomères éthyléniques, on préfère:

- l'éthylène, l'isoprène, et le butadiène ;
- l'acrylate de n-butyle, l'acrylate d'éthylhexyle, l'acrylate d'isobutyle, l'acrylate de n-hexyle
- le (méth)acrylate de méthoxyéthyle, le (méth)acrylate d'éthoxyéthyle, le (méth)acrylate POE (polyoxyéthylène avec répétition du motif oxyéthylène de 5 à 30 fois) et le (méth)acrylate POE - alkyl ($C_1$ à $C_{30}$) (POE) avec répétition du motif oxyéthylène de 5 à 30 fois) ;
- l'éther de vinyle et de méthyle, et l'éther de vinyle et d'éthyle ;
- le propionate de vinyle, le butyrate de vinyle, l'éthylhexanoate de vinyle, le néononanoate de vinyle, et le néododécanoate de vinyle
- le vinylcyclohexane, le styrène et l'acétate de vinyle,
- les acrylates de t-butylcyclohexyle, de tertiobutyle, de t-butylbenzyle, de furfuryle et d'isobornyle ;
- - les méthacrylates de méthyle, d'éthyle, de n-butyle, d'isobutyle, le méthacrylate de n-hexyle, de t-butylcyclohexyle, de t-butylbenzyle, de méthoxypropyle et d'isobornyle ;
- le N-butylacrylamide, le N-t-butylacrylamide, le N-isopropylacrylamide, le N,N-diméthylacrylamide et le N,N-dibutylacrylamide.

Parmi les monomères hydrophiles choisis encore plus préférentiellement, on peut citer:

- la vinylpyrrolidone, la vinylcaprolactame, l'acide (méth)acrylique, l'acide styrènesulfonique, l'acide acrylamidomethylpropanesulfonique, l'acide vinylbenzoïque, l'acide vinylphosphonique, ainsi que leur sels : Le neutralisant peut être une base minérale, telle que LiOH, NaOH, KOH, $Ca(OH)_2$, $NH_4OH$; ou une base organique, par exemple une amine primaire, secondaire ou tertiaire, notamment une alkylamine éventuellement hydroxylée comme la dibutylamine, la triéthylamine, la stearamine, ou bien l'amino-2-méthyl-2-propanol, la monoéthanolamine, la diéthanolamine, la stearamidopropyldimethylamine.

- les (méth)acrylates ou les (méth)acrylamides aminoalkyle en C1 à C6, N,N-dialkyle en C1 à C4 tel que Le méthacrylate de N,N-diméthylaminoéthyle (MADAME), le diméthylaminopropylmethacrylamide (DMAPMA) ainsi que leur sels ou formes quaternisées. Parmi les acides minéraux, on peut citer l'acide sulfurique ou l'acide chlorhydrique, Parmi les acides organiques, on peut citer les acides comportant un ou plusieurs groupes carboxylique, sulfonique, ou phosphonique : les acides acétique ou propionique, lactique, tartrique. Les agents quaternisants peuvent être des halogénures d'alkyles tel que bromure de méthyle ou des sulfates d'alkyle tels le methylsulfate ou la propane sultone.

Dans les compositions selon l'invention, le milieu cosmétiquement acceptable comprend, avantageusement, un ou plusieurs solvants appropriés choisis parmi l'eau, les cétones, les alcools, les alkylèneglycols, les éthers d'alkylèneglycol, les acétates d'alkyle en $C_{2-7}$, les éthers, les alcanes, les hydrocarbures aromatiques, les aldéhydes et les huiles volatiles.

Les compositions peuvent comprendre en outre un gaz propulseur, bien connu de l'homme de l'art dans le domaine des aérosols.

Dans le cas de certaines compositions cosmétiques, ledit milieu cosmétiquement acceptable comprend, en outre, avantageusement, une phase grasse composée de corps gras liquides ou solides à température ambiante, d'origine animale, végétale, minérale ou synthétique.

Il est possible, de manière avantageuse, que ledit milieu cosmétiquement acceptable comprenne en outre un ou plusieurs agents épaississants, un ou plusieurs polymères filmogènes anioniques, cationiques ou non ioniques synthétiques ou dérivés naturels et/ou un ou plusieurs agents plastifiants.

Dans certains cas encore, ledit milieu cosmétiquement acceptable comprend en outre une phase particulaire constituée de pigments et/ou de nacres et/ou de charges.

Par ailleurs, ledit milieu cosmétiquement acceptable peut, avantageusement, comprendre en outre un ou plusieurs additifs tels que des agents antioxydants, des parfums, des huiles essentielles, des conservateurs, des actifs cosmétiques lipophile ou hydrophiles, des agents hydratants, des vitamines, des colorants, des acides gras essentiels, des sphingolipides, des agents autobronzants, des filtres solaires, des agents anti-mousse, des agents séquestrants, des agents antioxydants ou des agents anti-radicalaires.

Les compositions cosmétiques selon l'invention se présentent, avantageusement, sous forme de lotion, de suspension, de dispersion, de solution organique, aqueuse ou hydroalcoolique éventuellement épaissie ou gélifiée, de mousse, de spray, d'aérosol, d'émulsion huile-dans-eau, eau-dans-huile ou multiple, de poudre libre, compacte ou coulée, de solide ou de pâte anhydre.

Avantageusement, les compositions cosmétiques selon l'invention forment une laque ou un produit de coiffage pour cheveux.

Un autre objet de l'invention concerne un procédé cosmétique, comprenant l'application d'une composition comprenant, dans un milieu cosmétiquement acceptable, au moins un polymère hyperbranché comprenant au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre, chaque point de ramifications étant disposé au coeur d'au moins une chaîne et comprenant au moins une séquence polymérique dont la température de transition vitreuse (Tg) est comprise entre -150 et -20°C, et de préférence, entre -150 et -30°C, et plus préférentiellement entre -150 et -40°C, la proportion relative en poids de cette séquence polymérique étant supérieure à 50 %, et préférentiellement supérieure à 55% et plus préférentiellement encore supérieure à 60%, par rapport au poids total du polymère.

On préfère tout particulièrement les polymères hyperbranchés présentant un point de ramification trivalent, ainsi que les compositions cosmétiques comprenant de tels polymères hyperbranchés, les procédés mettenat en oeuvre de tels polymères et leur utilisation en cosmétique, notamment capillaire.

Un objet particulièrement préféré de l'invention concerne l'utilisation d'un polymère hyperbranché comprenant au moins trois branches polymériques, formant indifféremment la branche principale ou une branche secondaire, et comportant, chacune, au moins un point de ramification trivalent pour former au moins deux points de ramification trivalents, distincts et indépendants l'un de l'autre, disposés au coeur de chaque chaîne, au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre, chaque point de ramifications étant disposé au coeur d'au moins une chaîne, comprenant au moins une séquence polymérique dont la température de transition vitreuse (Tg) est comprise entre -150 et -20°C, et de préférence, entre -150 et -30°C, et plus préférentiellement entre-150 et -40°C, la proportion relative en poids de cette séquence polymérique étant supérieure à 50 %, et préférentiellement supérieure à 55% et plus préférentiellement encore supérieure à 60%, par rapport au poids total du polymère, dans une composition destinée à la fixation et/ou au maintien de la coiffure.

L'invention pourra être mieux comprise à l'aide de l'exemple non limitatif qui suit et qui constitue un mode de réalisation préférentiel des polymères selon l'invention.

## Exemple 1:

Le polymère est synthétisé par polymérisation radicalaire en présence d'un amorceur radicalaire de type peroxide (tbutylperoxypivalate), d'allylméthacrylate de formule CH2=C(CH3)CO2CH2CH=CH2 , de monomères éthyléniques .

Dans un réacteur muni d'un agitateur et d'un système de condensation, est introduit le solvant. Le réacteur est chauffé au reflux du solvant 80°C.

L'addition des monomères se fait en deux étapes pour faire réagir de façon préférentielle le premier mélange (mélange

1) avant l'addition du deuxième mélange (mélange 2). En parallèle est additionné l'amorceur (solution 3). La température est maintenue au reflux (8°C), après la fin d'adition du mélange 2, la polymérisation est poursuivie.

L'addition des différents mélanges et solution se fait grâce à un système d'introduction contrôlée en fonction du temps.

Exemple 1 (conforme à l'invention):

| Solvant | Poids En g | % Isopropanol / poids total de solvant | % solvant / solvant + monomères |
|---|---|---|---|
| Isopropanol<br>Eau | 80 g<br>20g | 80% | 50% |

| | monomères | Poids en g | % dans mélange 1 | Tg théorique Séquence 1 |
|---|---|---|---|---|
| Mélange 1 | Acrylate de butyle | 65 | 79.8 | -30°C |
| | Acide acrylique | 15 | 18.5 | |
| | Allyle Méthacrylate | 1.4 | 1.7 | |

| | | Poids en g | % / poids total de monomères |
|---|---|---|---|
| Solution 3 | Amorceur : tbutylperoxypivalate | 0.7 g | 0.7 |
| | Solvant : iPROH / eau : 80/20 | 20 g | |

| | monomères | Poids en g | % dans mélange 2 | Tg théorique Séquence 2 |
|---|---|---|---|---|
| Mélange 2 | Acide acrylique | 20g | 99.25% | + 100°C |
| | Allyle Méthacrylate | 0.15 | | |

En récapitulatif :

| Monomères | % dans polymère |
|---|---|
| Acrylate de butyle | 65 |
| Acide acrylique | 35 |
| Allyle Méthacrylate | 1.5% |

A 100g du mélange de solvants (isopropanol 80/ eau 20), est additionné le mélange 1 sur 1h alors que l'introduction de la solution 3 est réalisée sur 4 h.
Est alors additionné le mélange 2 sur une période de 2h.
La température est maintenue au reflux (80°C), la réaction est poursuivie 2 h.
Le polymère est alors précipité dans l'éther de pétrole à raison de 200g de solution de polymère pour 500g de précipitant.
Le polymère est séché en étuve à 40°C.

Rendement 99%.

Le polymère est alors neutralisé par de l'amino2 méthyl2 propanol (AMP) à raison de 1 mol par mol d'acide.
Le polymère est alors dissous dans l'éthanol à raison de 6%.

**Exemple 2: (art antérieur - exemple du 3 du brevet NOVEON WO01/96429)**

On utilise le même procédé que pour l'exemple 1 mais avec les pourcentages suivants

| | monomères | Poids en g | % dans mélange 1 | Tg Séquence 1 |
|---|---|---|---|---|
| Mélange 1 | Acrylate de butyle | 180 | 89.8 | -17°C |
| | Acide Méthacrylique | 17.5 | 8.7 | |
| | Allyle Méthacrylate | 3 | 1.5 | |

| | | Poids en g | % par rapport poids total de monomères |
|---|---|---|---|
| Solution 3 | Amorceur : tbutylperoxypivalate | 2.52 g | 0.7 |
| | Solvant : iPROH / eau : 80 | | 0.7 |

| | monomères | Poids en g | % dans mélange 2 | Tg théorique Séquence 2 |
|---|---|---|---|---|
| Mélange 2 | Acide Méthacrylique | 158 | 99 | +145°C |
| | Allyle Méthacrylate | 1.5 | 1 | |
| 360g | | | | |

| Monomères | % dans polymère |
|---|---|
| Acrylate de butyle | 50 |
| Acide méthacrylique | 48.75 |
| Allyle Méthacrylate | 1.25 |

## Revendications

1. Polymère hyperbranché comprenant au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre, chaque point de ramifications étant disposé au coeur d'au moins une chaîne, **caractérisé par le fait qu'**il comprend au moins une séquence polymérique dont la température de transition vitreuse (Tg) est comprise entre -150 et -20°C, et de préférence, entre -150 et -30°C, et plus préférentiellement entre -150 et -40°C, la proportion relative en poids de cette séquence polymérique étant supérieure à 50 %, et préférentiellement supérieure à 55% et plus préférentiellement encore supérieure à 60%, par rapport au poids total du polymère.

2. Polymère selon la revendication 1, **caractérisé par le fait qu'**il comprend, en outre, au moins une séquence polymérique dont la température de transition vitreuse (Tg) est comprise entre 20 et 150°C. et préférentiellement entre 20 et 100°C.

3. Polymère selon l'une quelconque des revendications 1 ou 2, **caractérisé par le fait que** sa recouvrance instantanée ($R_i$), comprise entre 50 et 100 % de préférence comprise entre 55% et100% mieux encore entre 55 et 95% et idéalement entre 60et 95%.

4. Polymère selon l'une quelconque des revendications précédentes, **caractérisé par le fait que** sa recouvrance à 300 secondes ($R_{300}$), est comprise entre 55 et 100 %, de préférence comprise entre 60% et 100% mieux encore, entre 80 et 100%.

5. Polymère selon l'une quelconque des revendications précédentes, **caractérisé par le fait qu'**il présente une élongation à la rupture supérieure 1000 % et de préférence supérieure à 1500%.

6. Polymère selon l'une quelconque des revendications précédentes, **caractérisé par le fait qu'**il comprend des points de réticulation formant entre 0.1 et 10 %, et préférentiellement entre 0.5 et 5% du nombre total des liaisons du polymère.

7. Polymère selon l'une quelconque des revendications précédentes, **caractérisé par le fait qu'**il présente une solubilité dans l'eau supérieure à 1 % en poids à 20°C.

8. Polymère selon l'une quelconque des revendications précédentes, **caractérisé par le fait qu'**il comprend des motifs dérivés d'un ou de plusieurs monomères éthyléniques.

9. Polymère selon l'une quelconque des revendications précédentes, **caractérisé par le fait que** sa masse moléculaire moyenne en nombre du copolymère est comprise entre 5000 g/mol et 1 500 000 g/mol, notamment entre 5500 g/mol et 1000 000 g/mol, et encore mieux entre 6000 g/mol et 900 000 g/mol.

10. Polymère selon l'une quelconque des revendications précédentes, **caractérisé par le fait qu'**il comprend des motifs hydrophiles, dont la proportion en poids est comprise entre 5 et 70%, de préférence entre 5 et 65% et plus préférentiellement entre 5 et 50%, ces motifs hydrophiles étant, en particulier, dérivés d'un ou de plusieurs monomères éthyléniques.

11. Polymère selon la revendication 8 ou 10, **caractérisé par le fait que** les motifs dérivés d'un ou de plusieurs monomères éthyléniques sont choisis parmi les acides carboxyliques ou sulfoniques tel que l'acide acrylique ou méthacrylique, les (méth)acrylates d'alkyle en $C_{1-20}$ à chaîne linéaire, ramifiée ou cyclique ou hétérocyclique, les (méth)acrylates d'hydroxyalkyle en $C_{1-4}$, certains esters de vinyle , certains éthers de vinyle, le styrène, certains styrènes substitués, les monomères hétérocycliques, les (méth)acrylates de mono-, di- ou poly(éthylèneglycol) à extrémité hydroxyle éventuellement éthérifiée, le (méth)acrylamide, certains méthacrylamides aliphatiques, cycloaliphatiques ou aromatiques , les monomères (méth)acrylates ou vinyliques à groupe fluoré ou perfluoré ou les (méth) acrylamides à groupe fluoré ou perfluoré, les monomères (méth)acrylates ou vinyliques siliconés ou les (méth) acrylamides siliconés, les monomères acryliques ou vinyliques comportant une fonction amine éventuellement neutralisée ou quaternisée, et les dérivés carboxybétaïnes ou sulfobétaïnes éthyléniques.

12. Polymère selon l'une quelconque des revendications précédentes, **caractérisé par le fait que** le point de ramification est un point de ramification trivalent.

13. Procédé cosmétique, comprenant l'application d'une composition comprenant, dans un milieu cosmétiquement acceptable, au moins un polymère hyperbranché comprenant au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre, chaque point de ramifications étant disposé au coeur d'au moins une chaîne, et comprenant au moins une séquence polymérique dont la température de transition vitreuse (Tg) est comprise entre-150 et -20°C, et de préférence, entre-150 et -30°C, et plus préférentiellement entre -150 et -40°C, la proportion relative en poids de cette séquence polymérique étant supérieure à 50 %, et préférentiellement supérieure à 55% et plus préférentiellement encore supérieure à 60%, par rapport au poids total du polymère.

14. Composition cosmétique, **caractérisée par le fait qu'**elle comprend, dans un milieu cosmétiquement acceptable, au moins un polymère hyperbranché comprenant au moins trois branches polymériques, formant indifféremment la branche principale ou une branche secondaire, et comportant, chacune, au moins un point de ramification au moins trivalent pour former au moins deux points de ramification au moins trivalents, distincts et indépendants l'un de l'autre, chaque point de ramifications étant disposé au coeur d'au moins une chaîne, et ce polymère comprend au moins une séquence polymérique dont la température de transition vitreuse (Tg) est comprise entre -150 et -20°C, et de préférence, entre -150 et -30°C, et plus préférentiellement entre -150 et -40°C, la proportion relative en poids de cette séquence polymérique étant supérieure à 50 %, et préférentiellement supérieure à 55% et plus préférentiellement encore supérieure à 60%, par rapport au poids total du polymère.

15. Composition selon la revendication 14, **caractérisée par le fait que** le milieu cosmétiquement acceptable comprend un ou plusieurs solvants appropriés choisis parmi l'eau, les cétones, les alcools, les alkylèneglycols, les éthers

d'alkylèneglycol, les acétates d'alkyle en $C_{2-7}$, les éthers, les alcanes, les hydrocarbures aromatiques, les aldéhydes et les huiles volatiles.

**16.** Composition cosmétique selon l'une quelconque des revendications 14 à 15, **caractérisée par le fait que** ledit milieu cosmétiquement acceptable comprend en outre une phase grasse composée de corps gras liquides ou solides à température ambiante, d'origine animale, végétale, minérale ou synthétique.

**17.** Composition cosmétique selon l'une quelconque des revendications 14 à 16, **caractérisée par le fait que** ledit milieu cosmétiquement acceptable comprend, en outre, un ou plusieurs polymères filmogènes anioniques, cationiques ou non ioniques synthétiques ou dérivés naturels et/ou un ou plusieurs agents plastifiants.

**18.** Composition cosmétique selon l'une quelconque des revendications 14 à 17, **caractérisée par le fait que** ledit milieu cosmétiquement acceptable comprend en outre une phase particulaire constituée de pigments et/ou de nacres et/ou de charges.

**19.** Composition cosmétique selon l'une quelconque des revendications 14 à 18, **caractérisé par le fait que** ledit milieu physiologiquement acceptable comprend en outre un ou plusieurs additifs tels que des agents antioxydants, des parfums, des huiles essentielles, des conservateurs, des actifs cosmétiques lipophile ou hydrophiles, des agents hydratants, des vitamines, des colorants, des acides gras essentiels, des sphingolipides, des agents autobronzants, des filtres solaires, des agents anti-mousse, des agents séquestrants, des agents antioxydants ou des agents anti-radicalaires.

**20.** Composition cosmétique selon l'une quelconque des revendications 14 à 19, **caractérisée par le fait qu'**elle se présente sous forme de lotion, de suspension, de dispersion, de solution organique, aqueuse ou hydroalcoolique éventuellement épaissie ou gélifiée, de mousse, de spray, d'émulsion huile-dans-eau, eau-dans-huile ou multiple, de poudre libre, compacte ou coulée, de solide ou de pâte anhydre.

**21.** Composition cosmétique selon l'une quelconque des revendications 14 à 20, **caractérisée par le fait qu'**il s'agit d'une laque pour cheveux ou d'un produit de coiffage pour cheveux.

**Claims**

**1.** Hyperbranched polymer comprising at least three polymeric branches, forming either the main branch or a secondary branch and each having at least one at least trivalent branching point in order to form at least two at least trivalent branching points that are separate from and independent of each other, each branching point being located within the core of at least one chain, **characterized in that** it comprises at least one polymeric sequence whose glass transition temperature (Tg) is between -150 and -20°C, preferably between -150 and -30°C and more preferably between -150 and -40°C, the relative proportion by weight of this polymeric sequence being greater than 50%, preferably greater than 55% and even more preferably greater than 60% relative to the total weight of the polymer.

**2.** Polymer according to Claim 1, **characterized in that** it furthermore includes at least one polymeric sequence whose glass transition temperature (Tg) is between 20 and 150°C and preferably between 20 and 100°C.

**3.** Polymer according to either of Claims 1 and 2, **characterized in that** its instantaneous recovery ($R_i$) is between 50 and 100%, preferably between 55 and 100%, better still between 55 and 95% and ideally between 60 and 95%.

**4.** Polymer according to any one of the preceding claims, **characterized in that** its 300-second recovery ($R_{300}$) is between 55 and 100%, preferably between 60 and 100% and better still between 80 and 100%.

**5.** Polymer according to any one of the preceding claims, **characterized in that** it has an elongation at break of greater than 1000% and preferably greater than 1500%.

**6.** Polymer according to any one of the preceding claims, **characterized in that** it comprises crosslinking points forming between 0.1 and 10% and preferably between 0.5 and 5% of the total number of bonds of the polymer.

**7.** Polymer according to any one of the preceding claims, **characterized in that** it has a water solubility of greater than 1% by weight at 20°C.

**8.** Polymer according to any one of the preceding claims, **characterized in that** it comprises units derived from one or more ethylenic monomers.

**9.** Polymer according to any one of the preceding claims, **characterized in that** its number-average molecular weight of the copolymer is between 5000 g/mol and 1 500 000 g/mol, especially between 5500 g/mol and 1 000 000 g/mol and better still between 6000 g/mol and 900 000 g/mol.

**10.** Polymer according to any one of the preceding claims, **characterized in that** it comprises hydrophilic units, the proportion by weight of which is between 5 and 70%, preferably between 5 and 65% and more preferably between 5 and 50%, these hydrophilic units being, in particular, derived from one or more ethylenic monomers.

**11.** Polymer according to Claim 8 or 10, **characterized in that** the units derived from one or more ethylenic monomers are chosen from carboxylic or sulfonic acids, such as acrylic or methacrylic acid, $C_{1-20}$ alkyl (meth)acrylates with a linear, branched, cyclic or heterocyclic chain, $C_{1-4}$ hydroxyalkyl (meth)acrylates, certain vinyl esters, certain vinyl ethers, styrene, certain substituted styrenes, heterocyclic monomers, optionally etherified hydroxyl-terminated monoethylene glycol, diethylene glycol or polyethylene glycol (meth)acrylates, (meth)acrylamide, certain aliphatic, cycloaliphatic or aromatic methacrylamides, (meth)acrylate or vinyl monomers with a fluorinated or perfluorinated group or (meth)acrylamides with a fluorinated or perfluorinated group, silicone-containing vinyl or (meth)acrylate monomers or silicone-containing (meth)acrylamides, acrylic or vinyl monomers having an optionally neutralized or quaternized amine function, and ethylenic carboxybetaine or sulfobetaine derivatives.

**12.** Polymer according to any one of the preceding claims, **characterized in that** the branching point is a trivalent branching point.

**13.** Cosmetic method, comprising the application of a composition comprising, in a cosmetically acceptable medium, at least one hyperbranched polymer comprising at least three polymeric branches, forming either the main branch or a secondary branch and each having at least one at least trivalent branching point in order to form at least two at least trivalent branching points that are separate from and independent of each other, each branching point being located within the core of at least one chain, and comprising at least one polymeric sequence whose glass transition temperature (Tg) is between -150 and - 20°C, preferably between -150 and -30°C and more preferably between -150 and -40°C, the relative proportion by weight of this polymeric sequence being greater than 50%, preferably greater than 55% and even more preferably greater than 60% relative to the total weight of the polymer.

**14.** Cosmetic composition, **characterized in that** it comprises, in a cosmetically acceptable medium, at least one hyperbranched polymer comprising at least three polymeric branches, forming either the main branch or a secondary branch and each having at least one at least trivalent branching point in order to form at least two at least trivalent branching points that are separate from and independent of each other, each branching point being located within the core of at least one chain, and this polymer comprises at least one polymeric sequence whose glass transition temperature (Tg) is between -150 and -20°C, preferably between -150 and -30°C and more preferably between -150 and -40°C, the relative proportion by weight of this polymeric sequence being greater than 50%, preferably greater than 55% and even more preferably greater than 60% relative to the total weight of the polymer.

**15.** Composition according to Claim 14, **characterized in that** the cosmetically acceptable medium comprises one or more suitable solvents chosen from water, ketones, alcohols, alkylene glycols, alkylene glycol ethers, $C_{2-7}$ alkyl acetates, ethers, alkanes, aromatic hydrocarbons, aldehydes and volatile oils.

**16.** Cosmetic composition according to either of Claims 14 and 15, **characterized in that** said cosmetically acceptable medium furthermore includes a fatty phase composed of fatty substances that are liquid or solid at room temperature, of animal, plant, mineral or synthetic origin.

**17.** Cosmetic composition according to any one of Claims 14 to 16, **characterized in that** said cosmetically acceptable medium furthermore includes one or more anionic, cationic or nonionic, synthetic or naturally derived, film-forming polymers and/or one or more plasticizers.

**18.** Cosmetic composition according to any one of Claims 14 to 17, **characterized in that** said cosmetically acceptable medium furthermore includes a particulate phase consisting of pigments and/or nacres and/or fillers.

**19.** The cosmetic composition according to any one of Claims 14 to 18, **characterized in that** said physiologically

acceptable medium furthermore includes one or more additives such as antioxidants, perfumes, essential oils, preserving agents, lipophilic or hydrophilic cosmetic active substances, hydrating agents, vitamins, dyes, essential fatty acids, sphingolipids, self-tanning agents, sunscreens, antifoam agents, sequestrants, antioxidants or free-radical scavengers.

**20.** Cosmetic composition according to any one of Claims 14 to 19, **characterized in that** it is in the form of an organic, aqueous or hydroalcoholic lotion, suspension, dispersion or solution, optionally thickened or gelled, a mousse, a spray, an oil-in-water, water-in-oil or multiple emulsion, a free, compact or cast powder, a solid or an anhydrous paste.

**21.** Cosmetic composition according to any one of Claims 14 to 20, **characterized in that** it is a hair lacquer or a hairstyling product.

**Patentansprüche**

**1.** Hochverzweigtes Polymer, das mindestens drei Polymerzweige aufweist, die in gleicher Weise den Hauptzweig oder einen Nebenzweig bilden und jeder mindestens einen mindestens dreiwertigen Verzweigungspunkt aufweist, um mindestens zwei mindestens dreiwertige Verzweigungspunkte zu bilden, die unterschiedlich und voneinander unabhängig sind, wobei jeder Verzweigungspunkt im Innern mindestens einer Kette angeordnet ist, **dadurch gekennzeichnet, dass** es mindestens eine Polymersequenz aufweist, deren Glasübergangstemperatur (Tg) im Bereich von -150 bis -20 °C, vorzugsweise -150 bis -30 °C und besonders bevorzugt im Bereich von -150 bis -40 °C liegt, wobei der relative Gewichtsanteil dieser Polymersequenz größer 50 % und vorzugsweise größer 55 % und noch bevorzugter größer 60 %, bezogen auf das Gesamtgewicht des Polymers, ist.

**2.** Polymer nach Anspruch 1, **dadurch gekennzeichnet, dass** es ferner mindestens eine Polymersequenz aufweist, deren Glasübergangstemperatur (Tg) im Bereich von 20 bis 150 °C und vorzugsweise 20 bis 100 °C liegt.

**3.** Polymer nach einem der Ansprüche 1 oder 2, **dadurch gekennzeichnet, dass** seine augenblickliche Rückverformung ($R_i$) im Bereich von 50 bis 100 %, vorzugsweise 55 bis 100 %, besser 55 bis 95 % und idealerweise 60 bis 95 % liegt.

**4.** Polymer nach einem der vorhergehenden Ansprüche, **dadurch gekennzeichnet, dass** seine Rückverformung bei 300 Sekunden ($R_{300}$) im Bereich von 55 bis 100 %, vorzugsweise 60 bis 100 % und noch besser 80 bis 100 % liegt.

**5.** Polymer nach einem der vorhergehenden Ansprüche, **dadurch gekennzeichnet, dass** es eine Reißdehnung über 1 000 % und vorzugsweise über 1 500 % besitzt.

**6.** Polymer nach einem der vorhergehenden Ansprüche, **dadurch gekennzeichnet, dass** es Vernetzungspunkte aufweist, die 0,1 bis 10 % und vorzugsweise 0,5 bis 5 % der Gesamtzahl der Bindungen des Polymers ausmachen.

**7.** Polymer nach einem der vorhergehenden Ansprüche, **dadurch gekennzeichnet, dass** es bei 20 °C in Wasser eine Löslichkeit über 1 Gew.-% aufweist.

**8.** Polymer nach einem der vorhergehenden Ansprüche, **dadurch gekennzeichnet, dass** es Einheiten aufweist, die von einem oder mehreren ethylenischen Monomeren abgeleitet sind.

**9.** Polymer nach einem der vorhergehenden Ansprüche, **dadurch gekennzeichnet, dass** die zahlenmittlere Molmasse des Copolymers im Bereich von 5 000 bis 1 500 000 g/mol, insbesondere 5 500 bis 1 000 000 g/mol und besser 6 000 bis 900 000 g/mol liegt.

**10.** Polymer nach einem der vorhergehenden Ansprüche, **dadurch gekennzeichnet, dass** es hydrophile Einheiten enthält, deren Gewichtsanteil im Bereich von 5 bis 70 %, vorzugsweise 5 bis 65 % und noch bevorzugter 5 bis 50 % liegt, wobei diese hydrophilen Einheiten insbesondere von einem oder mehreren ethylenischen Monomeren abgeleitet sind.

**11.** Polymer nach Anspruch 8 oder 10, **dadurch gekennzeichnet, dass** die Einheiten, die von einem oder mehreren ethylenischen Monomeren abgeleitet sind, unter den Carbonsäuren oder Sulfonsäuren, wie Acrylsäure oder Methacrylsäure, Alkyl($C_{1-20}$) (meth)acrylaten mit gerader, verzweigter oder cyclischer oder heterocyclischer Kette,

Hydroxyalkyl($C_{1-4}$)(meth)acrylaten, bestimmten Vinylestern, bestimmten Vinylethern, Styrol, bestimmten substituierten Styrolen, heterocyclischen Monomeren, Mono-, Di- oder Poly(ethylenglycol)(meth)acrylaten mit gegebenenfalls verethertem Hydroxyende, (Meth)acrylamid, bestimmten aliphatischen, cycloaliphatischen oder aromatischen Methacrylamiden, (Meth)acrylatmonomeren oder Vinylmonomeren mit fluorierter oder perfluorierter Gruppe oder (Meth)acrylamiden mit fluorierter oder perfluorierter Gruppe, (Meth)acrylatmonomeren oder Vinylmonomeren, die siliconiert sind, oder siliconierten (Meth)acrylamiden, Acrylmonomeren oder Vinylmonomeren, die eine gegebenenfalls neutralisierte oder quaternisierte Aminofunktion aufweisen, und ethylenischen Carboxybetain- oder Sulfobetainderivaten ausgewählt sind.

**12.** Polymer nach einem der vorhergehenden Ansprüche, **dadurch gekennzeichnet, dass** der Vernetzungspunkt ein dreiwertiger Vernetzungspunkt ist.

**13.** Kosmetisches Verfahren, das das Aufbringen einer Zusammensetzung umfasst, die in einem kosmetisch akzeptablen Medium mindestens ein hochverzweigtes Polymer enthält, das mindestens drei Polymerzweige aufweist, die in gleicher Weise den Hauptzweig oder einen Nebenzweig bilden und jeder mindestens einen mindestens dreiwertigen Verzweigungspunkt aufweist, um mindestens zwei mindestens dreiwertige Verzweigungspunkte zu bilden, die unterschiedlich und voneinander unabhängig sind, wobei jeder Verzweigungspunkt im Innern mindestens einer Kette angeordnet ist, und mindestens eine Polymersequenz aufweist, deren Glasübergangstemperatur (Tg) im Bereich von -150 bis -20 °C, vorzugsweise -150 bis -30 °C und besonders bevorzugt im Bereich von -150 bis -40 °C liegt, wobei der relative Gewichtsanteil dieser Polymersequenz größer 50 % und vorzugsweise größer 55 % und noch bevorzugter größer 60 %, bezogen auf das Gesamtgewicht des Polymers, ist.

**14.** Kosmetische Zusammensetzung, **dadurch gekennzeichnet, dass** sie in einem kosmetisch akzeptablen Medium mindestens ein hochverzweigtes Polymer enthält, das mindestens drei Polymerzweige aufweist, die in gleicher Weise den Hauptzweig oder einen Nebenzweig bilden und jeder mindestens einen mindestens dreiwertigen Verzweigungspunkt aufweist, um mindestens zwei mindestens dreiwertige Verzweigungspunkte zu bilden, die unterschiedlich und voneinander unabhängig sind, wobei jeder Verzweigungspunkt im Innern mindestens einer Kette angeordnet ist, und das Polymer mindestens eine Polymersequenz aufweist, deren Glasübergangstemperatur (Tg) im Bereich von -150 bis -20°C, vorzugsweise -150 bis -30 °C und besonders bevorzugt im Bereich von -150 bis -40 °C liegt, wobei der relative Gewichtsanteil dieser Polymersequenz größer 50 % und vorzugsweise größer 55 % und noch bevorzugter größer 60 %, bezogen auf das Gesamtgewicht des Polymers, ist.

**15.** Zusammensetzung nach Anspruch 14, **dadurch gekennzeichnet, dass** das kosmetisch akzeptable Medium ein oder mehrere geeignete Lösungsmittel enthält, die unter Wasser, Ketonen, Alkoholen, Alkylenglycolen, Alkylenglycolethern, Alkyl($C_{2-7}$)acetaten, Alkanen, aromatischen Kohlenwasserstoffen, Aldehyden und flüchtigen Ölen ausgewählt sind.

**16.** Kosmetische Zusammensetzung nach einem der Ansprüche 14 bis 15, **dadurch gekennzeichnet, dass** das kosmetisch akzeptable Medium ferner eine Fettphase enthält, die aus bei Umgebungstemperatur flüssigen oder festen Fettsubstanzen tierischer; pflanzlicher, mineralischer oder synthetischer Herkunft zusammengesetzt ist.

**17.** Kosmetische Zusammensetzung nach einem der Ansprüche 14 bis 16, **dadurch gekennzeichnet, dass** das kosmetisch akzeptable Medium ferner ein oder mehrere anionische, kationische oder nichtionische filmbildende Polymere, die synthetisch oder von natürlichen Stoffen abgeleitet sind, und/oder einen oder mehrere Weichmacher enthält.

**18.** Kosmetische Zusammensetzung nach einem der Ansprüche 14 bis 17, **dadurch gekennzeichnet, dass** das kosmetisch akzeptable Medium ferner eine Partikelphase enthält, die aus Pigmenten und/oder Perlglanzpigmenten und/oder Füllstoffen besteht.

**19.** Kosmetische Zusammensetzung nach einem der Ansprüche 14 bis 18, **dadurch gekennzeichnet, dass** das physiologisch akzeptable Medium ferner ein oder mehre Additive enthält, wie Antioxidantien, Parfums, etherische Öle, Konservierungsmittel, lipophile oder hydrophile kosmetische Wirkstoffe, Hydratisierungsmittel, Vitamine, Farbmittel, essentielle Fettsäuren, Sphingolipide, Selbstbräunungsmittel, Sonnenschutzmittel, Schaumverhütungsmittel, Maskierungsmittel, Antioxidantien oder Radikalfänger für freie Radikale.

**20.** Kosmetische Zusammensetzung nach einem der Ansprüche 14 bis 19, **dadurch gekennzeichnet, dass** sie als Lotion, Suspension, Dispersion, organische, wässrige oder wässrig-alkoholische Lösung, die gegebenenfalls eingedickt oder geliert ist, Schaum, Spray, Öl-in-Wasser-Emulsion, Wasser-in-Öl-Emulsion oder multiple Emulsion, loses, kompaktiertes oder gegossenes Pulver, Feststoff oder wasserfreie Paste vorliegt.

**21.** Kosmetische Zusammensetzung nach einem der Ansprüche 14 bis 20, **dadurch gekennzeichnet, dass** es sich um einen Haarlack oder ein Produkt für die Frisurengestaltung für Haare handelt.

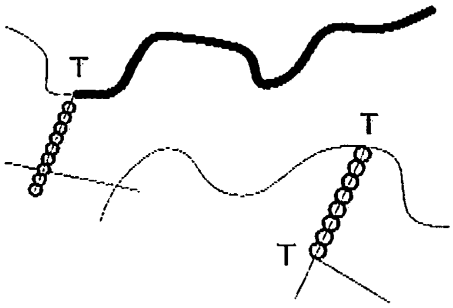


Fig. 1a

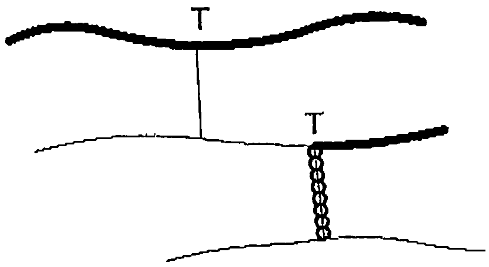


Fig. 1b

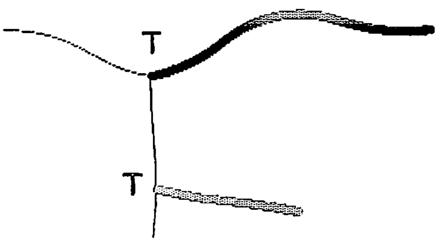


Fig. 1c

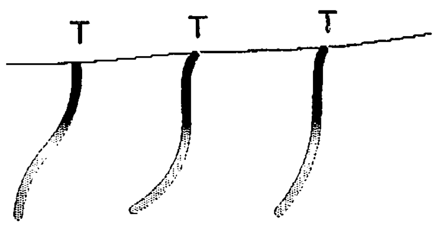


Fig. 1d

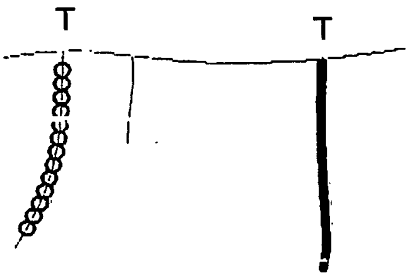


Fig. 1e

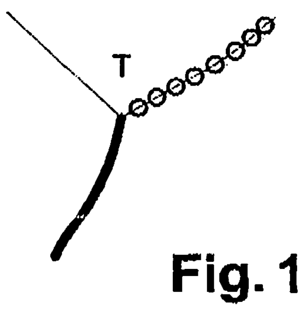


Fig. 1f

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 815848 A
- WO 9735541 A
- WO 0196429 A
- WO 9718247 A
- WO 9903894 A


### Littérature non-brevet citée dans la description


- *Glossary of basic terms in polymer science / IUPAC,* 1996
- **JHO JY ; YOO SH.** *macromolecules,* 2002, vol. 35, 1146-48
- *Polym prepr,* 1996, vol. 37 (2), 413-14
- *Matyjaszewski progress in polymer science,* 2001, vol. 26 (3), 337-77
- *Chem review,* 2001, vol. 101 (12), 3737
- *Mueller macromolecules,* 2001, vol. 34 (18), 62026-13
- **MATYJASEZWSKI et al.** *JACS,* 1995, vol. 117, 5614
- *Chem review,* 2001, vol. 101 (12), 3681
- Synthesis of nitroxy-functionalized polybutadiène by anionic polymerization using a nitroxy-functionalized terminator. *Macromolecules,* 1997, vol. 30, 4238-4242
- Macromolecular engineering via living free radical polymerizations. *Macromol. Chem. Phys.,* 1998, vol. 199, 923-935
- *chem review,* 2001, vol. 101 (12), 3787
- **CHARLEUX B ; R FAUST.** *Advances in polyme science,* 1999, vol. 142, 1-69